# EUROPEAN PATENT APPLICATION

(11) **EP 4 144 413 A1**
(43) Date of publication of application: **08.03.2023**
(21) Application number: 21779174.8
(22) Date of filing: 30.03.2021
(51) Int. Cl.: A61P 19/00, A61P 19/10, A23L 33/175, A61K 31/198

(54) **COMPOSITION FOR AMELIORATING OR PREVENTING DECREASES IN BONE STRENGTH**

(30) Priority: 30.03.2020 JP 2020060915
(71) Applicant: AJINOMOTO CO., INC., Chuo-ku Tokyo 104-8315 (JP)
(72) Inventor: SUSA, Yuko, Kawasaki-shi, Kanagawa 210-8681 (JP); ICHIKAWA, Reiko, Kawasaki-shi, Kanagawa 210-8681 (JP); INOUE, Yoshiko, Kawasaki-shi, Kanagawa 210-8681 (JP); TANAKA, Chie, Kawasaki-shi, Kanagawa 210-8681 (JP); SUZUKI, Katsuya, Kawasaki-shi, Kanagawa 210-8681 (JP)
(74) Representative: Strehl Schübel-Hopf & Partner
(86) International application number: PCT/JP2021/013624
(87) International publication number: WO 2021/200996

(57) **Abstract**

An object of the present invention is to provide a composition effective for preventing or improving bone strength decrease.

A composition for preventing or improving bone strength decrease (e.g., bone strength decrease due to bone density decrease, bone quality decrease, bone metabolism decrease, and the like), containing ornithine as an active ingredient.

## Description

### [Technical Field]

The present invention relates to a composition for preventing or improving bone strength decrease.

### [Background Art]

Osteoporosis is defined as a disease characterized by decreased bone strength, in which a risk of bone fracture increases, and this definition is generally used.

Bone strength is defined by two factors: bone density and bone quality. Bone density is determined by the amount of minerals such as calcium and the like constituting the bone (bone mass), and bone quality is defined by the material property which is the quality of the raw material of the bone, and the structural property (microstructure) built up from the material.

Since bone strength is defined by bone density and bone quality, if either of them decreases, the decrease in bone strengths and the risk of bone fracture increases. As one of the factors that decrease bone strength, it is known that accelerated bone resorption and decreased bone formation occur due to estrogen deficiency (menopause) and aging, which in turn leads to decreased bone density and deterioration of bone quality (Non Patent Literature 6).

In order to maintain the bone strength in a healthy state, a balance between bone formation by osteoblasts and bone resorption by osteoclasts (balance of bone metabolism) is important. It is known that one of the causes of osteoporosis is an imbalance in bone metabolism (bone resorption becomes stronger than bone formation) due to aging or menopause.

In addition, it is known that BMP-2 (Bone morphogenetic protein-2) signal, which is a differentiation factor into osteoblasts, decreases in elderly people, osteoporosis patients, and postmenopausal women (Non Patent Literatures 7 to 9).

It has been reported that ornithine is effective in improving liver function (Non Patent Literatures 1 and 2), reducing mental stress (Non Patent Literature 3), and recovering from fatigue (Non Patent Literatures 4 and 5).

However, the effect of ornithine on bone strength has not been reported to date.

### [Citation List]

### [Non Patent Literature]

[NPL 1]
   Hishida. Food Style 21 16(11) 87-9, 2012
[NPL 2]
   Tamai et al., Amino Acids, 45 (6), 1343-51, 2013
[NPL 3]
   Miyake et al., Nutrition Journal 13, 53, 2014
[NPL 4]
   Sugino et a1., Nutrition Research, 28 (11) 738-743, 2008
[NPL 5]
   Kokubo et al., BioPsychoSocial Medicine, 7, 6, 2013
[NPL 6]
   Guideline for prevention and treatment of osteoporosis 2015, Life Science Publishing Co., Ltd.
[NPL 7]
   Mehrunnisa M. Raje et al., Molecular Biology Reports (2019) 46: 1667-1674
[NPL 8]
   Fleet et al., Endocrinology, 137 (11), 4605, 1996
[NPL 9]
   Zhang et al., Exp Ther Med. 18(5), 3659-3666, 2019

### [Summary of Invention]

### [Technical Problem]

An object of the present invention is to provide a composition effective for preventing or improving bone strength decrease.

### [Solution to Problem]

The present inventors have conducted intensive studies and found that, as shown in the below-mentioned Experimental Examples 1 to 4, ornithine has an osteoblast differentiation promoting action, that the osteoblast differentiation promoting action of ornithine is exhibited even under low BMP-2 concentrations, and further that ornithine has an osteoclast differentiation suppressive action.

The present inventors have found from such new findings (osteoblast differentiation promoting action and osteoclast differentiation suppressive action of ornithine) that ornithine can balance bone metabolism by suppressing decrease in bone formation and promotion of bone resorption (see the above-mentioned Non Patent Literature 6), which are known factors that decrease bone strength, and can be effectively used to prevent or improve bone strength decrease.

In addition, the present inventors have found from such new findings (the osteoblast differentiation promoting action of ornithine is exhibited even under low BMP-2 concentrations) that ornithine can be effectively used to prevent or improve bone strength decrease also in elderly people, osteoporosis patients, and postmenopausal women with reduced BMP-2 signals.

The present inventors have further conducted intensive studies and found that, as shown in the below-mentioned Experimental Examples 5 to 8, ornithine has a bone density decrease suppressive effect in vivo, also promotes the expression of osteocalcin in vivo, suppresses muscle atrophy that occurs simultaneously with osteoporosis, suppresses the expression of muscle atrophy marker genes MuRF1 and Atg3, suppresses increase in liver weight, which is an indicator of fatty liver induced by OVX (ovariectomy), and suppresses the expression of SREBP1c, MTP, and PPARγ in the liver.

The present inventors have further studied based on these new findings, and completed the present invention.

Accordingly, the present invention provides the following.
[1] A composition for preventing or improving bone strength decrease, comprising ornithine as an active ingredient.
[2] The composition of the above-mentioned [1], wherein the bone strength decrease is caused by at least one selected from menopause, immobilization osteoporosis, disuse osteoporosis, and aging.
[3] The composition of the above-mentioned [1], wherein the bone strength decrease is caused by bone density decrease or bone quality decrease.
[4] The composition of the above-mentioned [3], wherein the bone quality decrease is caused by bone metabolism decrease.
[5] The composition of any of the above-mentioned [1] to [4], wherein the composition is an osteoblast differentiation promoter and/or an osteoclast differentiation inhibitor.
[6] The composition of any of the above-mentioned [1] to [5], wherein the composition is further for preventing or improving musculoskeletal function decrease.
[7] The composition of the above-mentioned [1], wherein the composition is for ingestion by a subject with a decreased BMP-2 signal.
[8] The composition of the above-mentioned [7], wherein the subject with a decreased BMP-2 signal is a postmenopausal woman, an osteoporosis patient, or an elderly person.
[9] A composition for inducing osteocalcin expression, comprising ornithine as an active ingredient.
[10] A composition for preventing or improving changes in physical condition due to changes in postmenopausal hormone balance, comprising ornithine as an active ingredient.
[11] The composition of the above-mentioned [10], wherein the change in the physical condition is caused by a bone strength decrease and/or a liver weight increase.
[12] The composition of any of the above-mentioned [1] to [11], wherein the composition is a food.
[13] The composition of the above-mentioned [9], imparted with an indication of a function exhibited by osteocalcin expression induction.
[14] The composition of the above-mentioned [13], wherein the indication of a function is selected from the group consisting of "prevention of osteoporosis", "strengthening bone", "promotion of regeneration of bone", "bone building capacity", "suppression of bone fracture", and "acceleration of recovery from bone fracture".
[15] A composition for suppressing the expression of MuRF1 and/or Atg3, comprising ornithine as an active ingredient.
[16] The composition of the above-mentioned [15], imparted with an indication of a function exhibited by suppressing the expression of MuRF1 and/or Atg3.
[17] The composition of the above-mentioned [16], wherein the indication of a function is selected from the group consisting of "maintaining body in movable state for a long time", "maintaining walking function", "maintaining exercise function", and "being able to walk for a long time".
[18] A composition for suppressing the expression of SREBPlc and/or MTP and/or PPARγ, comprising ornithine as an active ingredient.
[19] The composition of the above-mentioned [18], wherein the composition is imparted with an indication of a function exhibited by suppressing the expression of SREBP1c and/or MTP and/or PPARγ.
[20] A method for preventing or improving a bone strength decrease in a subject in need of the prevention or improvement of bone strength decrease, comprising administering a composition comprising an effective amount of ornithine to the subject.
[21] The method of the above-mentioned [20], wherein the bone strength decrease is caused by at least one selected from menopause, immobilization osteoporosis, disuse osteoporosis, and aging.
[22] The method of the above-mentioned [20], wherein the bone strength decrease is caused by bone density decrease or bone quality decrease.
[23] The method of the above-mentioned [22], wherein the bone quality decrease is caused by bone metabolism decrease.
[24] The method of any one of the above-mentioned [20] to [23], wherein the prevention or improvement of bone strength decrease is achieved by the promotion of osteoblast differentiation and/or suppression of osteoclast differentiation.
[25] The method of any one of the above-mentioned [20] to [24], wherein the administration subject is in further need of the prevention or improvement of musculoskeletal function decrease.
[26] The method of the above-mentioned [20], wherein the administration subject is a subject with a decreased BMP-2 signal.
[27] The method of the above-mentioned [26], wherein the subject with a decreased BMP-2 signal is a postmenopausal woman, an osteoporosis patient, or an elderly person.
[28] A method for inducing osteocalcin expression in a subject in need of the induction of osteocalcin expression, comprising administering a composition comprising an effective amount of ornithine to the subject.
[29] A method for preventing or improving changes in physical condition due to changes in postmenopausal hormone balance in a subject in need of the prevention or improvement of changes in physical condition due to changes in postmenopausal hormone balance, comprising administering a composition comprising an effective amount of ornithine to the subject.
[30] The method of the above-mentioned [29], wherein the change in the physical condition is caused by bone strength decrease and/or liver weight increase.
[31] The method of any one of the above-mentioned [20] to [30], wherein the composition is orally administered.
[32] A method for suppressing the expression of MuRFl and/or Atg3 in a subject in need of the suppression of the expression of MuRFl and/or Atg3, comprising administering a composition comprising an effective amount of ornithine to the subject.
[33] A method for suppressing the expression of SREBPlc and/or MTP and/or PPARγ in a subject in need of the suppression of the expression of SREBP1c and/or MTP and/or PPARγ, comprising administering a composition comprising an effective amount of ornithine to the subject.
[34] A composition comprising ornithine for use in the prevention or improvement of bone strength decrease.
[35] The composition of the above-mentioned [34], wherein the bone strength decrease is caused by at least one selected from menopause, immobilization osteoporosis, disuse osteoporosis, and aging.
[36] The composition of the above-mentioned [34], wherein the bone strength decrease is caused by bone density decrease or bone quality decrease.
[37] The composition of the above-mentioned [36], wherein the bone quality decrease is caused by bone metabolism decrease.
[38] The composition of any of the above-mentioned [34] to [37], wherein the prevention or improvement of bone strength decrease is caused by the promotion of osteoblast differentiation and/or the suppression of osteoclast differentiation.
[39] The composition of any of the above-mentioned [34] to [38], wherein the composition is further for use in the prevention or improvement of musculoskeletal function decrease.
[40] The composition of the above-mentioned [34], wherein the prevention or improvement of bone strength decrease is prevention or improvement of bone strength decrease in a subject with a decreased BMP-2 signal.
[41] The composition of the above-mentioned [40], wherein the subject with a decreased BMP-2 signal is a postmenopausal woman, an osteoporosis patient, or an elderly person.
[42] A composition comprising ornithine for use in the induction of osteocalcin expression.
[43] A composition comprising ornithine for use in the prevention or improvement of changes in physical condition due to changes in postmenopausal hormone balance.
[44] The composition of the above-mentioned [43], wherein the change in physical condition is caused by bone strength decrease and/or liver weight increase.
[45] The composition of any of the above-mentioned [34] to [44], wherein the composition is a food.
[46] A composition comprising ornithine for use in the suppression of the expression of MuRFl and/or Atg3.
[47] A composition comprising ornithine for use in the suppression of the expression of SREBP1c and/or MTP and/or PPARγ.
[48] Use of ornithine for the production of a composition for preventing or improving bone strength decrease.
[49] The use of the above-mentioned [48], wherein the bone strength decrease is caused by at least one selected from menopause, immobilization osteoporosis, disuse osteoporosis, and aging.
[50] The use of the above-mentioned [48], wherein the bone strength decrease is caused by bone density decrease or bone quality decrease.
[51] The use of the above-mentioned [50], wherein the bone quality decrease is caused by bone metabolism decrease.
[52] The use of any of the above-mentioned [48] to [5l], wherein the composition is an osteoblast differentiation promoter and/or an osteoclast differentiation inhibitor.
[53] The use of any of the above-mentioned [48] to [52], wherein the composition is further for use in the prevention or improvement of musculoskeletal function decrease.
[54] The use of the above-mentioned [48], wherein the prevention or improvement of bone strength decrease is prevention or improvement of bone strength decrease in a subject with a decreased BMP-2 signal.
[55] The use of the above-mentioned [54], wherein the subject with a decreased BMP-2 signal is a postmenopausal woman, an osteoporosis patient, or an elderly person.
[56] Use of ornithine for the production of a composition for inducing osteocalcin expression.
[57] Use of ornithine for the production of a composition for preventing or improving changes in physical condition due to changes in postmenopausal hormone balance.
[58] The use of the above-mentioned [57], wherein the change in physical condition is caused by bone strength decrease and/or liver weight increase.
[59] The use of any of the above-mentioned [48] to [58], wherein the composition is a food.
[60] Use of ornithine for the production of a composition for suppressing the expression of MuRFl and/or Atg3.
[61] Use of ornithine for the production of a composition for suppressing the expression of SREBP1c and/or MTP and/or PPARγ.

### [Advantageous Effects of Invention]

The composition of the present invention containing ornithine can be effectively used for the prevention or improvement of bone strength decrease, based on the osteoblast differentiation promoting action, and/or osteoclast differentiation suppressive action of ornithine. Particularly, since ornithine shows an osteoblast differentiation promoting action even at low BMP-2 concentrations, the composition containing ornithine of the present invention is advantageous in that it can be used effectively for the prevention or improvement of bone strength decrease even in elderly people, osteoporosis patients, and postmenopausal women, showing decreased BMP-2 signal.

The composition containing ornithine of the present invention can be effectively used for the prevention or improvement of musculoskeletal function decrease such as disuse muscle atrophy and the like in subjects with decreased bone strength such as osteoporosis and the like.

The composition containing ornithine of the present invention can be effectively used for the prevention or improvement of changes in physical condition (poor physical condition) due to changes in postmenopausal hormone balance, particularly, changes in physical condition (poor physical condition) caused by bone strength decrease and/or liver weight increase.

### [Brief Description of Drawings]

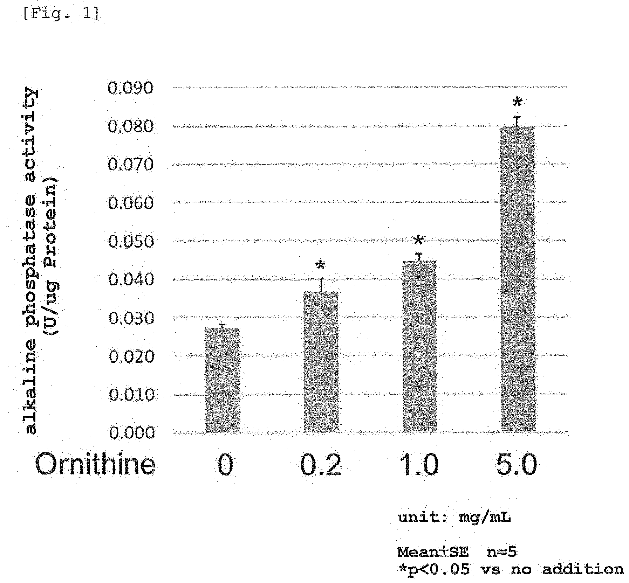

[Fig. l]
   Fig. 1 shows the results of Experimental Example 1.
[Fig. 2]

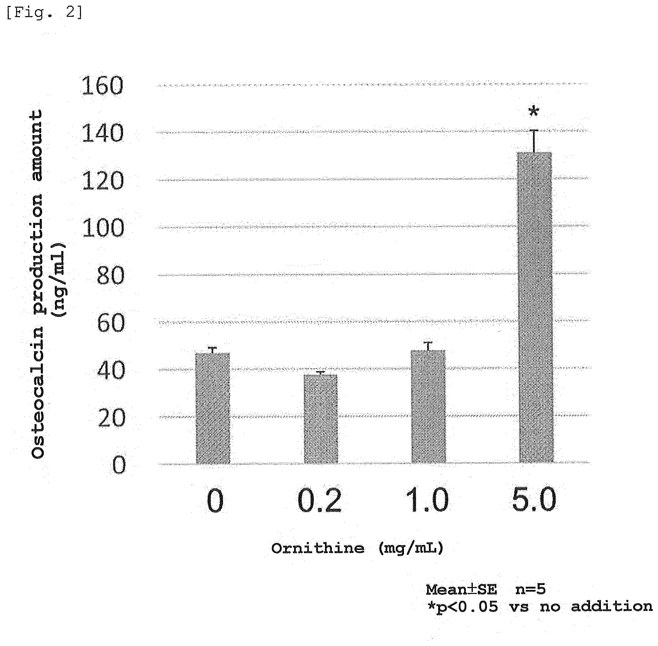

   Fig. 2 shows the results of Experimental Example 2.
[Fig. 3]

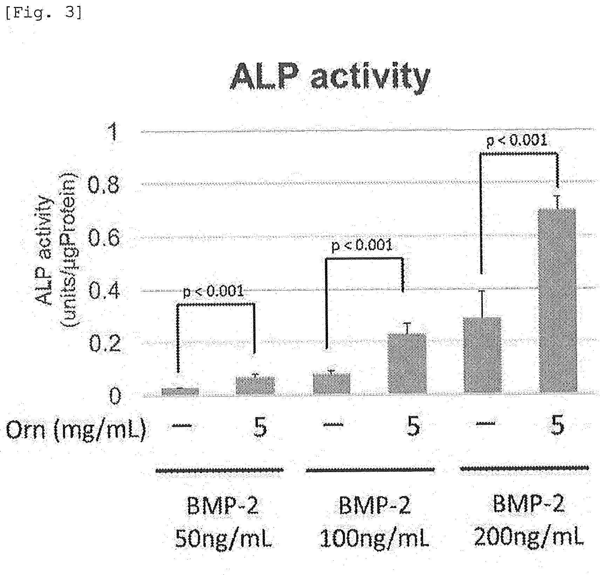

   Fig. 3 shows the results of Experimental Example 3. In Fig. 3, abbreviation Orn indicates ornithine.
[Fig. 4]

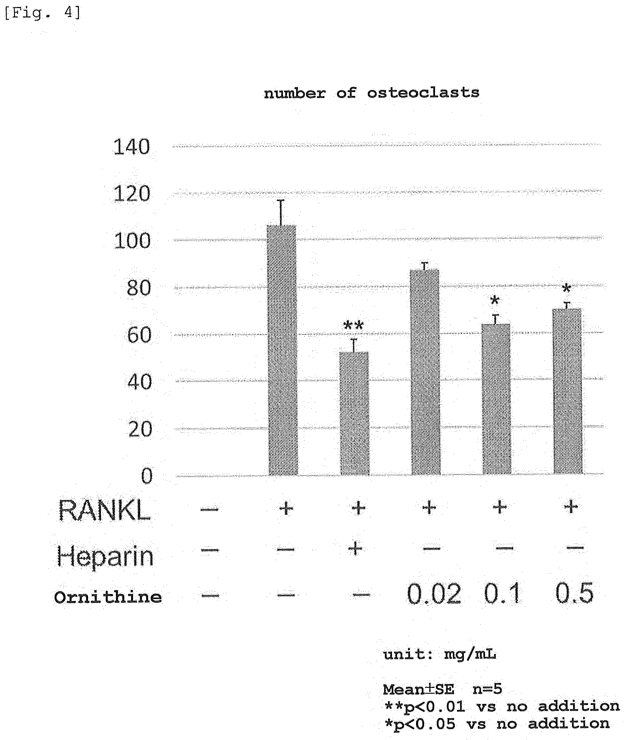

   Fig. 4 shows the results of Experimental Example 4.
[Fig. 5]

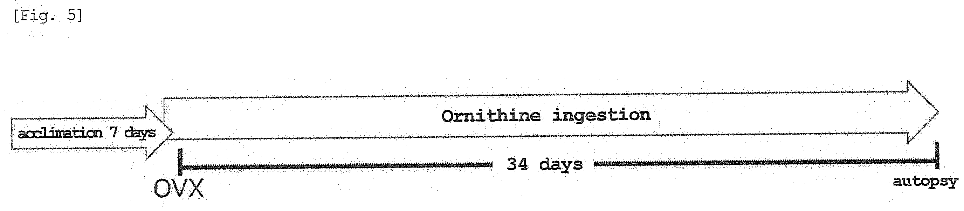

   Fig. 5 shows the experiment schedule for OVX (ornithine administration) group in Experimental Example 5.
[Fig. 6]

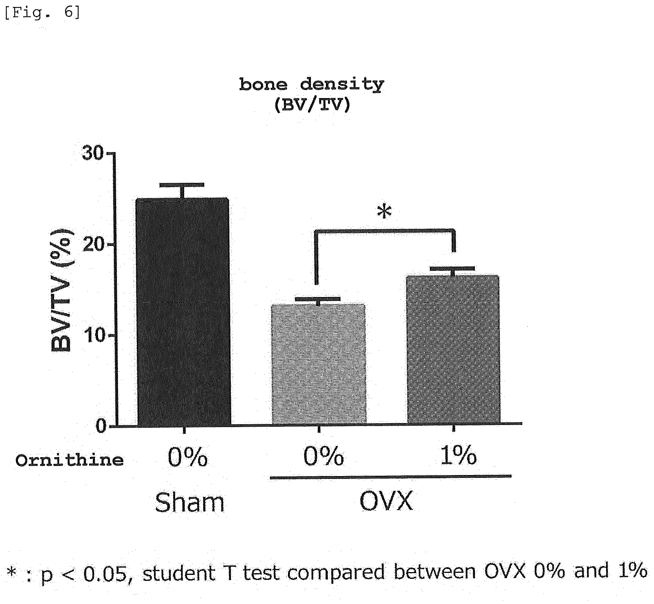

   Fig. 6 shows the results of Experimental Example 5.
[Fig. 7]

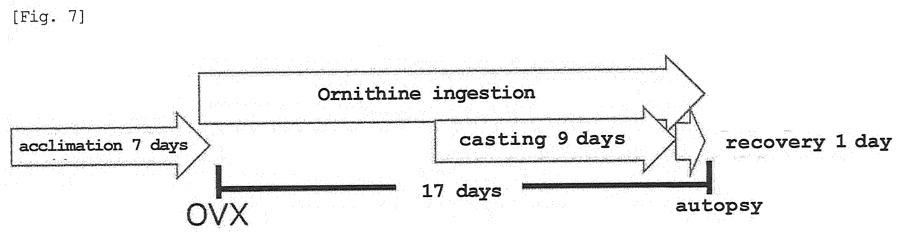

   Fig. 7 shows the experiment schedule for OVX (ornithine 1% blended feed administration with cast fixation) group and OVX (ornithine 3% blended feed administration with cast fixation) group in Experimental Example 6.
[Fig. 8]

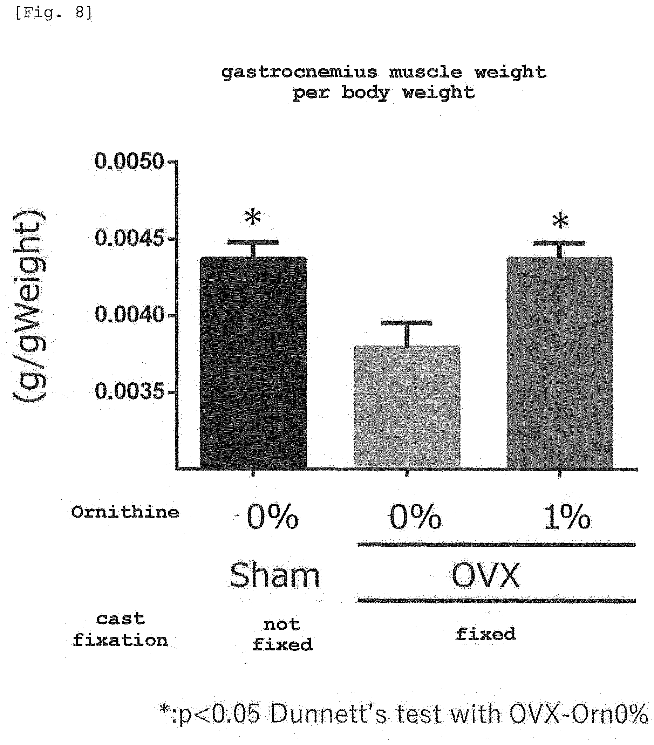

   Fig. 8 shows the results of Experimental Example 6.
[Fig. 9]

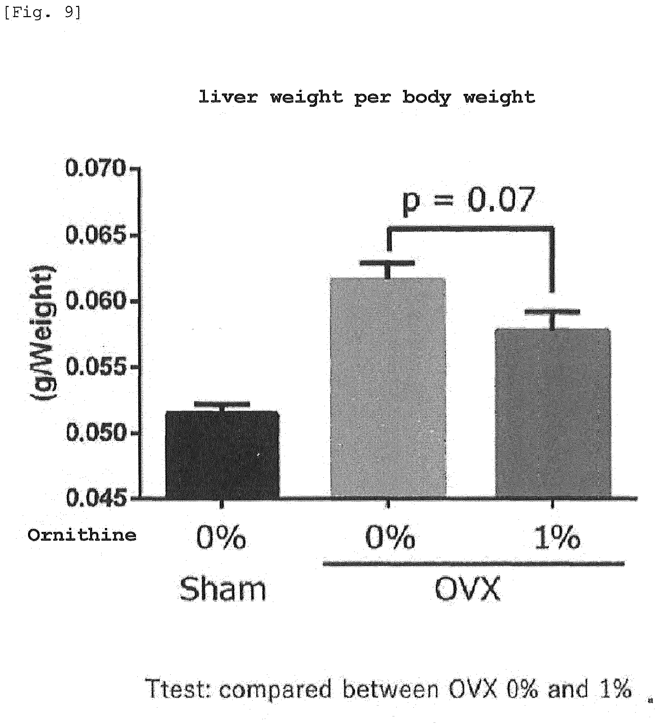

   Fig. 9 shows the results of Experimental Example 7.
[Fig. 10]

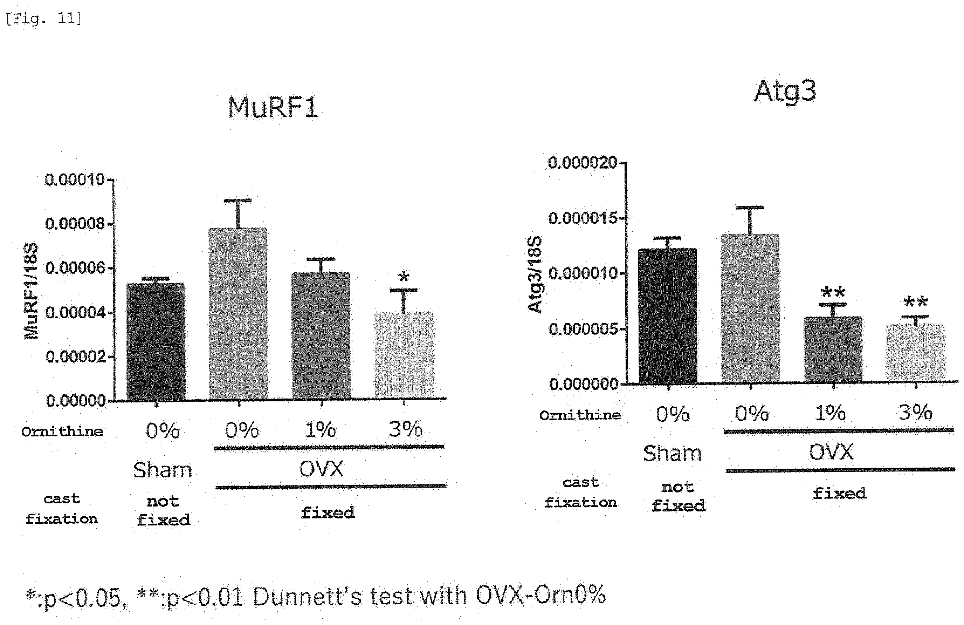

   Fig. 11 shows the results (osteocalcin expression level) of Example 8.
[Fig. 11]

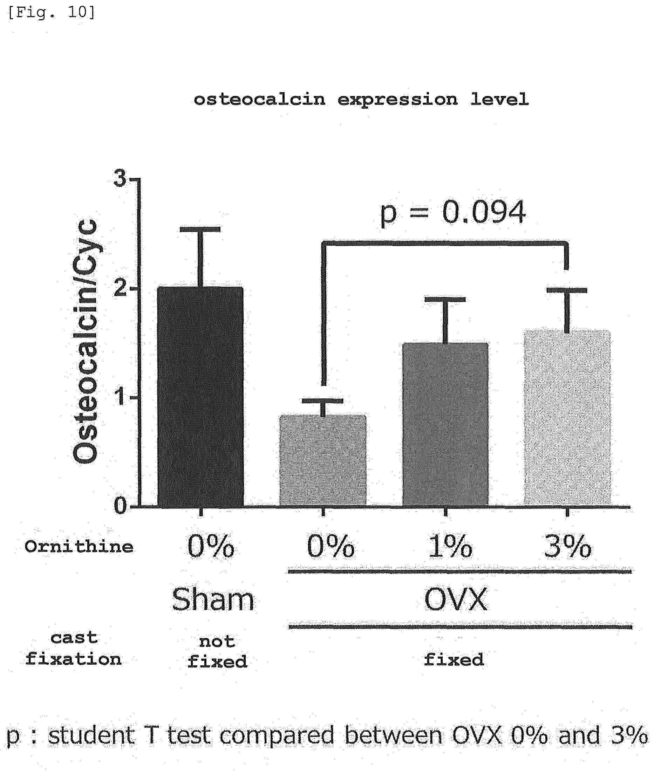

   Fig. 10 shows the results (MuRF1 and Atg3 expression level) of Example 8.
[Fig. 12-1]

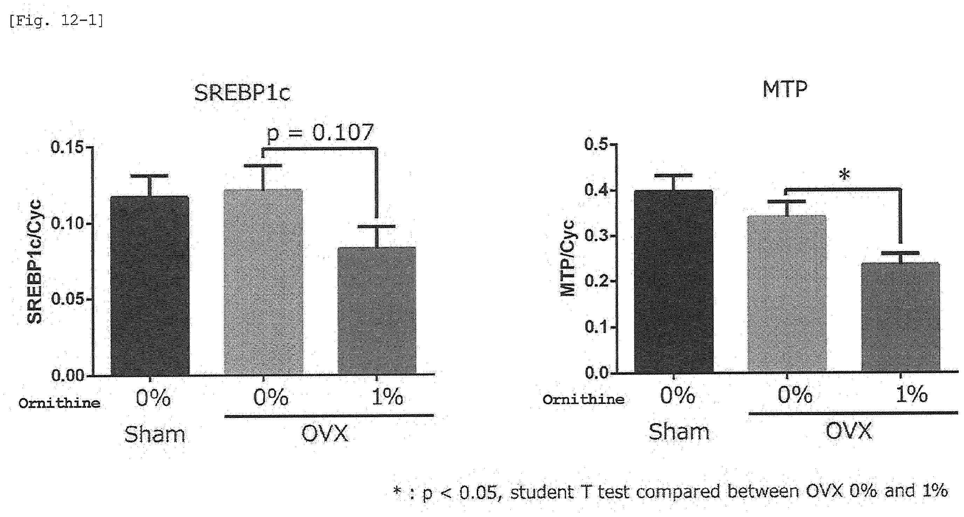

   Fig. 12-1 shows the results (SREBP1c and MTP expression levels) of Example 8.
[Fig. 12-21

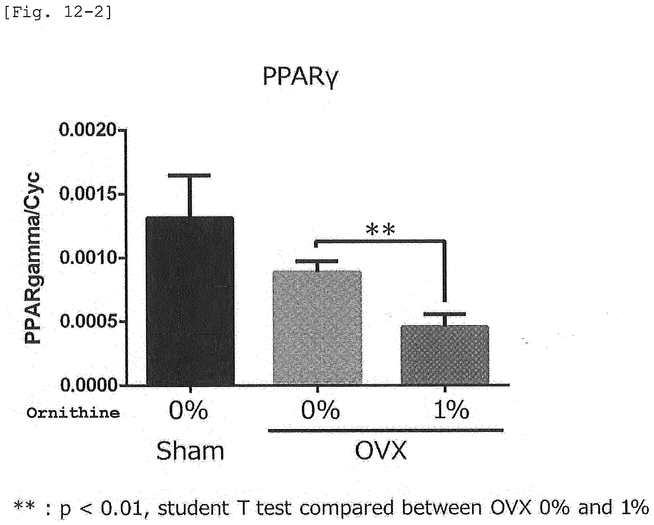

   Fig. 12-2 shows the results (PPARγ expression level) of Example 8.

### [Description of Embodiments]

The present invention is explained in detail in the following.

The composition for preventing or improving bone strength decrease of the present invention (hereinafter sometimes to be abbreviated as the composition of the present invention) contains ornithine as an active ingredient.

In the present invention, the "bone strength decrease" includes bone density (bone mass) decrease and bone quality decrease (e.g., bone metabolism decrease).

In the present invention, the "improvement of bone strength decrease" refers to bringing the decreased bone strength back to a healthy state or close to a healthy state, and the "prevention of bone strength decrease" refers to maintaining bone strength in a healthy state, or preventing further deterioration of the decreased bone strength.

In the present invention, the factors causing bone strength decrease are not particularly limited and include, for example, menopause, immobilization osteoporosis, disuse osteoporosis, aging, malnutrition (e.g., malnutrition in young women), young age (e.g., growth phase), bone strength decrease due to lack of exercise.

The composition of the present invention is particularly useful for bone strength decrease due to menopause, immobilization osteoporosis, disuse osteoporosis, and aging.

Particularly, the composition of the present invention is useful in that it can also be used for subjects with low BMP-2 signal (e.g., postmenopausal women, osteoporosis patients, elderly people).

The composition of the present invention can be used as an osteoblast differentiation promoter and/or an osteoclast differentiation inhibitor, since ornithine as the active ingredient shows an osteoblast differentiation promoting action, and an osteoclast differentiation suppressive action, as shown in the below-mentioned Experimental Examples.

The composition of the present invention can be formed as a composition with indication of a function.

Examples of the indication of a function include "prevention of osteoporosis", "strengthening bone", "promotion of regeneration of bone", "bone building capacity", "suppression of bone fracture", and "acceleration of recovery from bone fracture".

The composition for preventing or improving bone strength decrease of the present invention can be further used for preventing or improving musculoskeletal function decrease.

In the present invention, the "musculoskeletal function" includes a function of moving limbs and body and, for example, walking function can be mentioned.

In the present invention, the "musculoskeletal function decrease" includes musculoskeletal function decrease due to disuse muscle atrophy.

In the present invention, the "improvement of musculoskeletal function decrease" refers to bringing the decreased musculoskeletal function back to a healthy state or close to a healthy state, and the "prevention of musculoskeletal function decrease" refers to maintaining musculoskeletal function in a healthy state, or preventing further deterioration of the decreased musculoskeletal function.

The composition of the present invention is useful in that it can be expected to achieve both effects of prevention or improvement of bone strength decrease and prevention or improvement of musculoskeletal function decrease, by administration to subjects who have simultaneously developed bone strength decrease, such as osteoporosis and the like, and musculoskeletal function decrease, such as disuse muscle atrophy and the like.

The composition of the present invention can be formed as a composition with indication of a function.

Examples of the indication of a function include "maintaining body in movable state for a long time", "maintaining walking function", "maintaining exercise function", and "being able to walk for a long time".

While ornithine as the active ingredient of the present invention may be in any of L-form, D-form, and DL-form, L-form is preferred.

In the present invention, ornithine may be in the form of a salt. As the salt, a salt acceptable as a food or medicament can be mentioned. Examples thereof include alkali metal salts such as sodium salt, potassium salt, and the like; alkaline earth metal salts such as calcium salt, magnesium salt, barium salt, and the like; aluminum salt; salts with organic bases such as ethylenediamine, propylenediamine, ethanolamine, monoalkyl ethanolamine, dialkyl ethanolamine, diethanolamine, triethanolamine, and the like; salts with inorganic acids such as hydrochloric acid, hydrobromic acid, nitric acid, sulfuric acid, phosphoric acid, and the like; and salts with organic acids such as formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

The content of ornithine contained in the composition of the present invention is, for example, 0.01 - 100 wt%, preferably 0.1 - 95 wt%, more preferably 1 - 90 wt%, further preferably 5 - 80 wt%.

While the composition of the present invention can be used as a food, a medicament, and the like, food is preferred.

In the present specification, food is a concept that broadly includes foods that can be ingested orally (excluding pharmaceutical products), and includes not only so-called "food" but also beverages, health supplement, food with health claims (e.g., food for specified health uses, food with functional claims), supplement, and the like.

The form of the composition of the present invention is not particularly questioned and may be, for example, powder, granule (including fine granule), tablet, hard capsule, soft capsule, liquid (e.g., solution, suspension, emulsion), drink, jelly, pudding, yogurt, candy, chewing gum, or the like. These can be produced by a known method. For example, the composition of the present invention is mixed with carriers (e.g., excipient, binder, disintegrant, lubricant, solvent) and powder, granule, tablet, capsule, liquid, and the like can be produced by a method known in the field of food preparation or pharmaceutical preparation. In addition, the composition of the present invention can also be produced by adding and mixing to and with food and drink (e.g., water, soft drink).

The ingestion amount (dose) of ornithine in the composition of the present invention is, for example, 1 mg to 24 g, preferably 50 mg to 24 g, more preferably 100 mg to 12 g, further preferably 200 mg to 4.8 g, per day for an adult (body weight 60 kg).

The composition of the present invention can be safely ingested by (administered to), human, animals other than human (e.g., mammals and birds such as domestic animals, poultry, experiment animals). The form of ingestion (administration) for animals other than human may be addition to a feed.

In one embodiment, the present invention relates to a composition for inducing osteocalcin expression, containing ornithine as an active ingredient.

In the composition for inducing osteocalcin expression of the present invention, the definition of ornithine, the content of ornithine, availability for food, medicament, and the like, form, ingestion amount, subject of ingestion and administration, and the like are the same as the definition of ornithine, the content of ornithine, availability for food, medicament, and the like, form, ingestion amount, subject of ingestion and administration, and the like in the above-mentioned composition for preventing or improving bone strength decrease of the present invention.

The osteocalcin expression induction can be confirmed, for example, by a method according to the below-mentioned Experimental Example 8.

The composition of the present invention can be expected to achieve effects such as "prevention of osteoporosis", "strengthening bone", "promotion of regeneration of bone", "bone building capacity", "suppression of bone fracture", "acceleration of recovery from bone fracture", and the like since ornithine as the active ingredient has an osteocalcin expression inducing action.

The composition for inducing osteocalcin expression of the present invention can be formed as a composition with indication of a function exhibited by induction of osteocalcin expression.

Examples of the indication of a function include "prevention of osteoporosis", "strengthening bone", "promotion of regeneration of bone", "bone building capacity", "suppression of bone fracture", and "acceleration of recovery from bone fracture".

In one embodiment, the present invention relates to a composition for suppressing the expression of MuRF1 and/or Atg3, containing ornithine as an active ingredient.

In the composition for suppressing the expression of MuRF1 and/or Atg3 of the present invention, the definition of ornithine, the content of ornithine, availability for food, medicament, and the like, form, ingestion amount, subject of ingestion and administration, and the like are the same as the definition of ornithine, the content of ornithine, availability for food, medicament, and the like, form, ingestion amount, subject of ingestion and administration, and the like in the above-mentioned composition for preventing or improving bone strength decrease of the present invention.

The suppression of the expression of MuRF1 and Atg3 can be confirmed, for example, by a method according to the below-mentioned Experimental Example 8.

The composition of the present invention can be expected to achieve effects such as "maintaining body in movable state for a long time", "maintaining walking function", "maintaining exercise function", "being able to walk for a long time" and the like since ornithine as the active ingredient has a suppressive action on the expression of MuRF1 and Atg3.

The composition for suppressing the expression of MuRF1 and/or Atg3 of the present invention can be formed as a composition with indication of a function exhibited by suppressing the expression of MuRF1 and/or Atg3.

Examples of the indication of a function include "maintaining body in movable state for a long time", "maintaining walking function", "maintaining exercise function", and "being able to walk for a long time".

In one embodiment, the present invention relates to a composition for suppressing the expression of SREBP1c and/or MTP and/or PPARγ, containing ornithine as an active ingredient.

In the composition for suppressing the expression of SREBP1c and/or MTP and/or PPARγ of the present invention, the definition of ornithine, the content of ornithine, availability for food, medicament, and the like, form, ingestion amount, subject of ingestion and administration, and the like are the same as the definition of ornithine, the content of ornithine, availability for food, medicament, and the like, form, ingestion amount, subject of ingestion and administration, and the like in the above-mentioned composition for preventing or improving bone strength decrease of the present invention.

The suppression of the expression of SREBP1c, MTP, and PPARγ can be confirmed, for example, by a method according to the below-mentioned Experimental Example 8.

The composition of the present invention can be expected to achieve effects such as improvement of fatty liver and hyperlipidemia, and the like since ornithine as the active ingredient has a suppressive action on the expression of SREBP1c, MTP, and PPARγ.

The composition for suppressing the expression of SREBP1c and/or MTP and/or PPARγ of the present invention can be formed as a composition with indication of a function exhibited by suppressing the expression of SREBP1c and/or MTP and/or PPARγ.

In one embodiment, the present invention relates to a composition for preventing or improving changes in physical condition due to changes in postmenopausal hormone balance, containing ornithine as an active ingredient.

As the changes in physical condition due to changes in postmenopausal hormone balance, changes in physical condition caused by decreased bone strength and/or increased liver weight can be mentioned. Specific examples of the changes in physical condition include bone fracture, decreased exercise, muscle atrophy associated with decreased exercise and decreased bone density, fatty liver, and hyperlipidemia.

In the composition for preventing or improving changes in physical condition due to changes in postmenopausal hormone balance of the present invention, the definition of ornithine, the content of ornithine, availability for food, medicament, and the like, form, ingestion amount, subject of ingestion and administration, and the like are the same as the definition of ornithine, the content of ornithine, availability for food, medicament, and the like, form, ingestion amount, subject of ingestion and administration, and the like in the above-mentioned composition for preventing or improving bone strength decrease of the present invention.

The composition of the present invention can be formed as a composition with indication of a function.

Examples of the indication of a function include "supporting changes in women's physical condition" and "improving poor physical condition in postmenopausal women".

### [Example]

The present invention is explained in more detail in the following by referring to Experimental Examples. The present invention is not limited to these.

### [Experimental Example l] Alkaline phosphatase (osteoblast differentiation marker) activity measurement test after ornithine addition, by using osteoprogenitor cell MC3T3-E1

Using the following materials and methods, osteoprogenitor cells were cultured in ornithine-added or ornithine-free medium, the alkaline phosphatase activity in the cultured cells was measured, and the osteoblast differentiation-promoting action of ornithine was examined.

### Materials

(1) Cell
   MC3T3-E1 (RIKEN Cell Bank, RCB1126)
(2) Medium
   α-MEM medium, 10% FBS, antibiotic added
(3) Test reagent, etc.
   α-MEM medium (phenol red free) (Invitrogen, Cat. No. 41061-029) Penicillin-streptomycin solution (nacalai tesque, Cat. No. 26253-84)
   0.25% trypsin-EDTA solution (nacalai tesque, Cat. No. 32777-44) Dulbecco's PBS (Nissui Pharmaceutical Co., Ltd., Code No. 05913)
   viable cell count measurement reagent SF (nacalai tesque, Cat. No. 07553-44)
   BMP-2, Human Recombinant (R&D Systems, Cat. No. 355-BEC-010) Micro BCA Protein Assay Reagent Kit (PIERCE, Cat. No. 23235) Cell-LyEX1 (Wako, Cat. No. 300-34761)
   LabAssay ALP (Wako, Cat. No. 291-58601)
   Mouse Osteocalcin EIA kit (Biomedical Technologies, Cat. No. BT-470)
   L-Ornithine Monohydrochloride (Nacalai, Cat. No. 25718-92)

### Method

### (1) Cell preculture

The cells were placed in a T-75 flask using a medium and cultured in a CO₂ incubator (5% CO₂, 37°C). The medium was changed every other day, and the cells were collected when they reached 80% confluence and used for the test.

### (2) Test

The cells were adjusted with medium to 1.2×10⁵ cells/0.2 mL/well and seeded in a 48-well plate. The next day, the medium was replaced with a medium supplemented with ornithine 0.2 mg/mL, 1.0 mg/mL or 5.0 mg/mL or additive-free medium, and the cells were cultured for 7 and 14 days. After culturing for 7 days, the cells were washed once with PBS for ALP activity measurement and then cryopreserved together with the plate. In addition, after culturing for 14 days, the culture supernatant was sampled for measurement of the amount of osteocalcin used in the below-mentioned Experimental Example 2, and cryopreserved. The test was performed with n=5 and the medium was changed every 3-4 days.

### (3) Alkaline phosphatase (ALP) activity measurement

The cells were washed with PBS and lysed with 60 µL/well of cell lysing agent (Cell-LyEX1 containing 2 mM PMSF). After stirring the plate at room temperature for 30 min, the supernatant was diluted 5-fold and the obtained solution was used as a sample for measurement. ALP activity in the cells was measured by LabAssay ALP. With this kit, ALP activity was measured from the amount of p-nitrophenol per unit amount of protein produced within a given period of time. The amount of protein in the solution was measured using Micro BCA Protein Assay Reagent Kit.

### (4) Significant difference test

The test was a two-sided test with Student's t-test, and P<0.05 (less than 5% of null hypothesis) or more was judged as significant difference. The data in the graph is shown in mean±standard error.

As shown in Fig. 1, alkaline phosphatase activity significantly increased by the addition of ornithine at 0.2 mg/mL, 1.0 mg/mL, and 5.0 mg/mL, as compared with no addition of ornithine.

The results show that ornithine promotes differentiation of osteoblasts.

### [Experimental Example 2] Osteocalcin (osteoblast differentiation marker) production amount measurement test after ornithine addition, by using osteoprogenitor cell MC3T3-E1

Using the "culture supernatant for measurement of the amount of osteocalcin" sampled in "method (2) test" of the above-mentioned Experimental Example 1, the osteocalcin (osteoblast differentiation marker) production amount was measured by the following method, and the osteoblast differentiation promoting action of ornithine was investigated.

### Method

### (1) Osteocalcin amount measurement

For the measurement of the amount of osteocalcin in the culture supernatant, the supernatant was diluted 10-fold and the measurement was performed using Mouse Osteocalcin EIA kit.

### (2) Significant difference test

The test was a two-sided test with Student's t-test, and P<0.05 (less than 5% of null hypothesis) or more was judged as significant difference. The data in the graph is shown in mean±standard error.

As shown in Fig. 2, osteocalcin production amount significantly increased by the addition of ornithine at 5.0 mg/mL, as compared with no addition of ornithine.

The results show that ornithine promotes differentiation of osteoblasts.

### [Experimental Example 3] Alkaline phosphatase (osteoblast differentiation marker) activity measurement test after ornithine addition, by using osteoprogenitor cell MC3T3-El (influence of BMP-2 (differentiation factor) concentration)

Using the following methods, osteoprogenitor cells were cultured in an ornithine-added or ornithine-free medium with varying BMP-2 (differentiation factor) concentrations and, the alkaline phosphatase activity in the cultured cells was measured, and the influence of BMP-2 (differentiation factor) concentration on the osteoblast differentiation-promoting action of ornithine was examined.

### Method

### (1) Cell preculture

The cells were placed in a 10 cm petri dish using a medium and cultured in a CO₂ incubator (5% CO₂, 37°C). The medium was changed every other day, and the cells were collected when they reached 80% confluence and used for the test.

### (2) Test

The cells were adjusted with medium to 1.2×10⁵ cells/0.2 mL/well and seeded in a 48-well plate. The next day, the condition was changed to a medium containing BMP-2 at 50 ng/mL, 100 ng/mL, or 200 ng/mL and free of ornithine or containing 5 mg/mL ornithine, and the cells were cultured for 7 days. After 7 days, the cells were washed once with PBS, 50 µL of 0.1% Triton-X was added, and the mixture was further sonicated for 1 min to prepare a cell lysate. The test was performed with n=4 and the medium was changed every 3-4 days.

### (3) Alkaline phosphatase (ALP) activity measurement

ALP activity of the above-mentioned cell lysate was measured by LabAssay ALP. With this kit, ALP activity was measured from the amount of p-nitrophenol per unit amount of protein produced within a given period of time. The amount of protein in the solution was measured using Micro BCA Protein Assay Reagent Kit.

### (4) Significant difference test

The test was a two-sided test with Student's t-test, and P<0.05 (less than 5% of null hypothesis) or more was judged as significant difference. The data in the graph is shown in mean±standard error.

As shown in Fig. 3, alkaline phosphatase activity was significantly increased by BMP-2 concentrations of 50 ng/mL, 100 ng/mL, and 200 ng/mL, and ornithine 5 mg/mL addition, as compared with ornithine no addition, irrespective of the BMP-2 addition concentration.

The results show that, while MC3T3-E1 cells differentiate into osteoblasts in a BMP-2 (differentiation factor) concentration-dependent manner, ornithine promotes differentiation of osteoblasts to the same level as that by the addition of BMP-2 at higher concentrations, even under low BMP-2 concentration (e.g., BMP-2 concentration 50 ng/mL) conditions.

### [Experimental Example 4] Osteoclast number measurement test after ornithine addition, by using osteoclast progenitor cell RAW264

Using the following materials and methods, osteoclast progenitor cells were cultured in ornithine-added or ornithine-free medium. After culture, the number of differentiated osteoclasts was measured, and the osteoclast number differentiation suppressive action of ornithine was examined.

### Material

(1) Cell
   RAW264 (Riken Cell Bank, RCB0535, lot40)
(2) Medium used
   proliferation medium: α-MEM medium, 10% FBS, antibiotic added test medium: α--MEM medium, 5% FBS, antibiotic added
(3) Test reagent, etc.
   α-MEM medium (phenol red free) (Invitrogen, Cat. No. 41061-029) Penicillin-streptomycin solution (nacalai tesque, Cat. No. 26253-84)
   Dulbecco's PBS (Nissui Pharmaceutical Co., Ltd., Cat. No. 05913)
   Cell Count Reagent SF (nacalai tesque, Cat. No. 07553-44) heparin (SIGMA, Cat. No. H3149-25KU)
   RANK ligand, Mouse, Recombinant (SIGMA, Cat. No. R0525-10UG) TRAP Activity Assay Kit (Immunodaiagnostic Systems, Cat. No. TR103)
   L-Ornithine Monohydrochloride (Nacalai, Cat. No. 25718-92)

### Method

### (1) Cell preculture

The cells were placed in a T-75 flask using a medium and cultured in a CO₂ incubator (5% CO₂, 37°C). The medium was changed every other day, and the cells were collected when they reached 80% confluence and used for the test.

### (2) Osteoclast differentiation test

Cells were prepared in a test medium at 3×10³ cells/0.1 mL/well and seeded in a 96 well plate. The next day, the medium was replaced with a 50 ng/mL RANK ligand (hereinafter to be abbreviated as RANKL) and ornithine 0.02 mg/mL, 0.1 mg/mL, or 0.5 mg/mL addition medium, a non-addition medium, a 100 µg/mL heparin addition medium as positive control, and a RANKL non-addition medium. After culturing for 7 days, TRAP staining was performed, and TRAP staining-positive and multinucleated cells were counted, based on which the ability to suppress differentiation into osteoclasts was examined by comparison. The medium was changed every 3-4 days. The test was performed with n=5.

### (3) Significant difference test

The test was a two-sided test with Student's t-test, and P<0.05 (less than 5% of null hypothesis) or more was judged as significant difference. The data in the graph is shown in mean±standard error.

As shown in Fig. 4, as compared with ornithine non-addition (in Fig. 4, the fifth bar graph from the right), the number of osteoclasts significantly decreased by the addition of ornithine 0.1 mg/mL and 0.5 mg/mL, and the number of osteoclasts decreased by the addition of ornithine 0.02 mg/mL.

The results show that ornithine suppresses differentiation of osteoclasts.

### [Experimental Example 5] Verification of bone density decrease suppressive effect of ornithine, using osteoporosis mouse model

To investigate the influence of ornithine on osteoporosis, the bone density decrease suppressive effect of ornithine was evaluated using an osteoporosis model prepared by ovariectomy (OVX) in mice according to the experiment method shown in the following.

### (Animal)

As the mouse, female, 8-week-old Balb/cA mice purchased from Charles River Laboratories Japan, Inc. were used. breeding conditions in breeding room:
temperature setting: 23°C±1°C
humidity setting: 60%±5%
Lighting time: 07:00-19:00 light period 19:00-07:00 dark period
Feeding: free food ingestion of CRF-l (ORIENTAL YEAST CO., LTD.)
Water supply: free drinking of tap water

### (Preparation of ornithine blended feed)

For ingestion of ornithine by mouse, a ornithine blended feed was produced. The composition was based on AIN93G, and was designed such that the total calorie was the same for all compositions by adjusting the amount of corn starch. Each composition is shown in Table 1. The production was performed at room temperature, and all materials were sufficiently mixed with a stirrer and stored in a cool dark place until use.

Each numerical value in Table 1 indicates the blended amount (g) per 1000 g of ornithine blended feed.

**[Table 1]**

| | ornithine 0% | ornithine 1% | ornithine 3% |
|---|---|---|---|
| vitamin-free casein (ORIENTAL YEAST CO., LTD.) | 200 | 200 | 200 |
| L-cystine (AJINOMOTO CO., INC.) | 1.8 | 1.8 | 1.8 |
| corn starch | 405.7 | 395.7 | 375.7 |
| α-starch (pregelatinized starch) (ORIENTAL YEAST CO., LTD.) | 155 | 155 | 155 |
| powdered sugar (sucrose) | 100 | 100 | 100 |
| cellulose (Toyo Roshi Kaisha, Ltd.) | 50 | 50 | 50 |
| soybean | 40 | 40 | 40 |
| t-butylhydroquinone (FUJIFILM) | 0.008 | 0.008 | 0.008 |
| mineral mix (ORIENTAL YEAST CO., LTD.) | 35 | 35 | 35 |
| AIN93 vitamin mix (ORIENTAL YEAST CO., LTD.) | 10 | 10 | 10 |
| choline hydrogen tartrate (FUJIFILM) | 2.5 | 2.5 | 2.5 |
| L(+)-ornithine monohydrochloride (NACALAI TESQUE, INC.) | 0 | 10 | 30 |
| Total | 1000 | 1000 | 1000 |

| | | | |
|---|---|---|---|
| amount blended per 1000 g of feed, unit: g | | | |

### (OVX surgical method)

(i) The hair on the back was shaved under isoflurane anesthesia, and an incision of about 8 mm was made around just below the kidney.
(ii) The peritoneum was also incised in the same manner, and the ovary was extracted from the body together with the adipose tissue exposed just below the kidney.
(iii) The upper and lower portions of the ovary were ligated, the ovary was completely removed, and sutured. This was performed on both sides.

The mice that had undergone successful ovariectomy began to gain weight in about one week after the operation, and their body weight increased significantly by the time of autopsy one month later. Therefore, the body weight was monitored after the operation. In addition, since the uterus atrophies significantly after ovariectomy, the success or failure of OVX was determined by measuring the weight of the uterus at autopsy.

In the present specification, the group of mice that underwent ovariectomy (OVX) is referred to as the OVX group. For comparison (control), a group of mice subjected to only shaving, incision of skin and peritoneum, and suturing as sham surgery, without ovariectomy is referred to as Sham group.

### (Experiment method)

As shown in Table 2, a comparative test was performed for the Sham (ornithine non-administration) group, the OVX (ornithine non-administration) group, and the OVX (ornithine administration) group.

In the OVX (ornithine administration) group, according to the experiment schedule shown in Fig.5, the diet was switched to l% ornithine blended feed from 3 days before the OVX surgery, ornithine was provided until immediately before autopsy, and autopsy was performed on the 34th day after OVX.

In the OVX (ornithine non-administration) group, the experiment was conducted in the same manner as in the OVX (ornithine administration) group, except that an ornithine 0% blended feed was ingested instead of the ornithine 1% blended feed.

In the Sham (ornithine non-administration) group, the experiment was conducted in the same manner as in the OVX (ornithine administration) group, except that the ornithine 0% blended feed was ingested instead of the ornithine 1% blended feed, and sham surgery was performed instead of OVX.

**[Table 2]**

| group | n | ornithine blended in feed |
|---|---|---|
| Sham (ornithine non-administration) group | 8 | ornithine 0% |
| OVX (ornithine non-administration) group | 8 | ornithine 0% |
| OVX (ornithine administration) group | 8 | ornithine 1% |

### (Measurement of bone density (BV/TV))

Bone density was measured using femur removed at autopsy. The removed femur was stored at -20°C until measurement. A micro CT device (Bruker, SKYSCAN) was used to measure bone density. The lower part of the femur was photographed, image was reconstructed (Bruker/NRecon), the bone angle was adjusted, 50 images were cut off from the growth plate reference section, and then 101 images were analyzed (Bruker, DataViewer/CTAn). The area of sponge bone was set and BV/TV was calculated. The results are shown in Fig. 6.

As shown in Fig. 6, in the OVX (ornithine non-administration) group (second bar graph from the left), bone density decreased as compared with the Sham (ornithine non-administration) group (first bar graph from the left). In the OVX (ornithine administration) group (third bar graph from the left), bone density significantly increased as compared with the OVX (ornithine non-administration) group.

From the results, it was confirmed that ornithine significantly suppresses bone density decrease due to OVX. In addition, a bone density decrease suppressive effect in the body by ornithine was confirmed.

### [Experimental Example 6] Verification of muscle atrophy suppressive effect of ornithine, using osteoporosis-disuse muscle atrophy simultaneous onset mouse model

To verify the effect of ornithine on disuse muscle atrophy that develops simultaneously with osteoporosis, osteoporosis due to OVX and disuse muscle atrophy due to fixation of the lower leg with cast were simultaneously developed in mice according to the experiment method shown below. Using the model, the muscle atrophy suppressive effect of ornithine was evaluated. Production of ornithine blended feed and OVX operation method were the same as those in Experimental Example 5.

### (Animal)

As the mouse, female, 8-week-old Balb/cA mice purchased from Charles River Laboratories Japan, Inc. were used. breeding conditions in breeding room:
temperature setting: 23°C±l°C
humidity setting: 60%±5%
Lighting time: 07:00-l9:00 light period 19:00-07:00 dark period
Feeding: free food ingestion of AIN93G (AJINOMOTO CO., INC., powder)
Water supply: free drinking of tap water

### (Method of cast fixation of lower leg)

(i) The second joint of the lower right leg of the mouse was bent under isoflurane anesthesia and lightly fixed with a paper adhesive tape or the like.
(ii) The thigh was wrapped around with a 1 cm wide binding tape (TRUSCO, Magic Band (R) Binding Tape (double-sided type)), and fixed by wrapping around the binding tape with another binding tape to completely cover the lower leg.
(iii) Fixation was performed for 9 days, and the tape was removed thereafter.

### (Experiment method)

As shown in Table 3, a comparative test was performed for the Sham (ornithine non-administration and no cast fixation) group, the OVX (ornithine non-administration with cast fixation) group, the OVX (ornithine 1% blended feed administration with cast fixation) group, and the OVX (ornithine 3% blended feed administration with cast fixation) group.

In the OVX (ornithine 1% blended feed administration with cast fixation) group and the OVX (ornithine 3% blended feed administration with cast fixation) group, according to the experiment schedule shown in Fig. 7, the diet was switched to 1% ornithine blended feed or 3% ornithine blended feed from 3 days before the OVX surgery, and ornithine was provided until immediately before autopsy. Cast fixation was applied for 9 days, after which a recovery period of about 24 hr was provided, and then autopsy was performed.

In the OVX (ornithine non-administration with cast fixation) group, the experiment was conducted in the same manner as in the OVX (ornithine 1% blended feed administration with cast fixation) group and the OVX (ornithine 3% blended feed administration with cast fixation) group, except that an ornithine 0% blended feed was ingested instead of the ornithine blended feed.

In the Sham (ornithine non-administration and no cast fixation) group, the experiment was conducted in the same manner as in the OVX (ornithine 1% blended feed administration with cast fixation) group and the OVX (ornithine 3% blended feed administration with cast fixation) group, except that the ornithine 0% blended feed was ingested instead of the ornithine blended feed, sham surgery was performed instead of OVX, and cast fixation was not performed.

**[Table 3]**

| group | cast fixation | n | ornithine blended in feed |
|---|---|---|---|
| Sham (ornithine non-administration with no cast fixation) group | not fixed | 8 | ornithine 0% |
| OVX (ornithine non-administration with cast fixation) group | fixed | 8 | ornithine 0% |
| OVX (ornithine 1% blended feed administration with cast fixation) group | fixed | 8 | ornithine 1% |
| OVX (ornithine 3% blended feed administration with cast fixation) group | fixed | 8 | ornithine 3% |

### (Measurement of gastrocnemius muscle weight)

The weight of the gastrocnemius muscle of the Sham (ornithine non-administration with no cast fixation) group, the OVX (ornithine non-administration with cast fixation) group, and the OVX (ornithine 1% blended feed administration with cast fixation) group, which was removed at autopsy, was measured, and the weight per body weight was calculated. The results are shown in Fig. 8.

As shown in Fig. 8, in the OVX (ornithine non-administration with cast fixation) group (second bar graph from the left), gastrocnemius muscle weight decreased as compared with the Sham (ornithine non-administration with no cast fixation) group (first bar graph from the left). In the OVX (ornithine 1% blended feed administration with cast fixation) group (third bar graph from the left), the gastrocnemius muscle weight increased as compared with the OVX (ornithine non-administration with cast fixation) group.

From the results, it was confirmed that ornithine suppresses muscle atrophy simultaneously developed with osteoporosis.

### [Experimental Example 7] Verification of liver weight suppressive effect of ornithine, using osteoporosis mouse model

To investigate the influence of ornithine on fatty liver and the like, the weight of the liver of the Sham (ornithine non-administration) group, the OVX (ornithine non-administration) group, and the OVX (ornithine administration) group in Experimental Example 5, which was removed at autopsy, was measured, and the liver weight per body weight was compared. The results are shown in Fig. 9.

As shown in Fig. 9, in the OVX (ornithine non-administration) group (second bar graph from the left), liver weight per body weight increased as compared with the Sham (ornithine non-administration) group (first bar graph from the left). In the OVX (ornithine administration) group (third bar graph from the left), liver weight decreased as compared with the OVX (ornithine non-administration) group.

The results show that ornithine suppresses liver weight increase which is an index of fatty liver induced by OVX.

### [Experimental Example 8] Gene expression analysis

To investigate the influence of ornithine on lifestyle-related disease-related factors, the gene expression analysis of the femur and gastrocnemius muscle removed at autopsy in Experimental Example 6, and the liver removed at autopsy in Experimental Example 5 was performed.

Muscle and the like were detached from the femur after removal, and the femur was immediately frozen in liquid nitrogen. The gastrocnemius muscle and the liver were also similarly frozen in liquid nitrogen immediately after removal. Using Multi-beads Shocker (YASUI KIKAI), the samples were pulverized, and RNA was extracted by a conventional method (NIPPON GENE CO., LTD./ISOGEN II). Using the extracted RNA, reverse transcription was performed, and cDNA was acquired (Takara Bio Inc./PrimeScript 1^{st}strand cDNA Synthesis Kit). Using the cDNA, the expression levels of osteocalcin (osteoblast marker) and cyclophilin A (housekeeping gene) were analyzed for the femur (Thermo Fisher SCIENTIFIC/QuantStudio 3, TaqMan Fast Advanced Master Mix). As for the gastrocnemius muscle, muscle atrophy markers MuRF1 and Atg3, and 18S (housekeeping gene) were measured. For the liver, SREBP1c, MTP, and PPARγ, which are the indices of lifestyle-related diseases, and cyclophilin A (housekeeping gene) were measured. The list of primers is shown in Table 4.

**[Table 4]**

| Real-time primer base sequence | | |
|---|---|---|
| Gene | Forward primer | Reverse primer |
| Osteocalcin | ACGGTATCACTATTTAGGACCTGTG (SEQ ID NO:1) | ACTTTATTTTGGAGCTGCTGTGAC (SEQ ID NO:2) |
| Cyclophilin A | TGGAGAGCACCAAGACAGACA (SEQ ID NO:3) | TGCCGGAGTCGACAATGAT (SEQ ID NO:4) |
| MuRF1 | GCTGGTGGAAAACATCATTGACA (SEQ ID NO:5) | CATCGGGGTGGCTGCCTTT (SEQ ID NO:6) |
| Atg3 | CTGGAGATCACTTAGTCCACCA (SEQ ID NO:7) | GTCGGAAGATATGCCTTCACTTT (SEQ ID NO:8) |
| 18S | AACGCCACTTGTCCCTCTAA (SEQ ID NO:9) | GTGGAGCGATTTGTCTGGTT (SEQ ID NO:10) |
| SREBP1c | GCCATGGATTGCACATTTG (SEQ ID NO:11) | TCAGGAGAGTTGGCACCTG (SEQ ID NO:12) |
| MTP | CCAGCATGATCCTCTTGGCA (SEQ ID NO:13) | TGAGAGGCCAGTTGTGTGAC (SEQ ID NO:14) |
| PPARγ | CGCTGATGCACTGCCTATGA (SEQ ID NO:15) | CGAGTGGTCTTCcATCACGG (SEQ ID NO:16) |

### (Statistical analysis)

The data was shown graphically using mean and SE values, and Student's t test was used for comparison of two groups and Dunnett test was used for comparison of three or more groups. For the analysis, Prism6 for Windows (GraphPad) was used. The results are shown in Fig. 10, Fig. 11, Fig. 12-1, and Fig. 12-2.

As shown in Fig. 10, in the OVX (ornithine non-administration with cast fixation) group (second bar graph from the left), osteocalcin expression level decreased as compared with the Sham (ornithine non-administration with no cast fixation) group (first bar graph from the left). In the OVX (ornithine 1% blended feed administration with cast fixation) group (third bar graph from the left) and the OVX (ornithine 3% blended feed administration with cast fixation) group (fourth bar graph from the left), the osteocalcin expression level increased as compared with the OVX (ornithine non-administration with cast fixation) group.

The results show that ornithine has an osteocalcin expression inducing action in vivo as well.

As shown in the left figure and the right figure in Fig. 11, in the OVX (ornithine non-administration with cast fixation) group (second bar graph from the left), the expression levels of MuRF1 and Atg3 increased as compared with the Sham (ornithine non-administration with no cast fixation) group (first bar graph from the left). However, in the OVX (ornithine 1% blended feed administration with cast fixation) group (third bar graph from the left) and the OVX (ornithine 3% blended feed administration with cast fixation) group (fourth bar graph from the left), the expression levels of MuRF1 and Atg3 decreased as compared with the OVX (ornithine non-administration with cast fixation) group.

The results show that ornithine has an action of suppressing the expression of MuRF1 and Atg3.

As shown in the left figure and the right figure in Fig. 12-1, and Fig. 12-2, in the OVX (ornithine administration) group (third bar graph from the left), the expression levels of SREBP1c, MTP, and PPARγ decreased as compared with the OVX (ornithine non-administration) group (second bar graph from the left) .

The results show that ornithine has an action of suppressing the expression of SREBP1c, MTP, and PPARγ in the liver.

### [Industrial Applicability]

According to the present invention, a composition effective for preventing or improving bone strength decrease and the like can be provided.

This application is based on patent application No. 2020-060915 filed in Japan, the contents of which are incorporated in full herein.

## Claims

1. A composition for preventing or improving bone strength decrease, comprising ornithine as an active ingredient.

2. The composition according to claim 1, wherein the bone strength decrease is caused by at least one selected from menopause, immobilization osteoporosis, disuse osteoporosis, and aging.

3. The composition according to claim 1, wherein the bone strength decrease is caused by bone density decrease or bone quality decrease.

4. The composition according to claim 3, wherein the bone quality decrease is caused by bone metabolism decrease.

5. The composition according to any one of claims 1 to 4, wherein the composition is an osteoblast differentiation promoter and/or an osteoclast differentiation inhibitor.

6. The composition according to any one of claims 1 to 5, wherein the composition is further for preventing or improving musculoskeletal function decrease.

7. The composition according to claim 1, wherein the composition is for ingestion by a subject with a decreased BMP-2 signal.

8. The composition according to claim 7, wherein the subject with a decreased BMP-2 signal is a postmenopausal woman, an osteoporosis patient, or an elderly person.

9. A composition for inducing osteocalcin expression, comprising ornithine as an active ingredient.

10. A composition for preventing or improving changes in physical condition due to changes in postmenopausal hormone balance, comprising ornithine as an active ingredient.

11. The composition according to claim 10, wherein the change in the physical condition is caused by a bone strength decrease and/or a liver weight increase.

12. The composition according to any one of claims 1 to 11, wherein the composition is a food.

13. The composition according to claim 9, imparted with an indication of a function exhibited by osteocalcin expression induction.

14. The composition according to claim 13, wherein the indication of a function is selected from the group consisting of "prevention of osteoporosis", "strengthening bone", "promotion of regeneration of bone", "bone building capacity", "suppression of bone fracture", and "acceleration of recovery from bone fracture".

15. A composition for suppressing the expression of MuRFl and/or Atg3, comprising ornithine as an active ingredient.

16. The composition according to claim 15, imparted with an indication of a function exhibited by suppressing the expression of MuRF1 and/or Atg3.

17. The composition according to claim 16, wherein the indication of a function is selected from the group consisting of "maintaining body in movable state for a long time", "maintaining walking function", "maintaining exercise function", and "being able to walk for a long time".

18. A composition for suppressing the expression of SREBP1c and/or MTP and/or PPARγ, comprising ornithine as an active ingredient.

19. The composition according to claim 18, wherein the composition is imparted with an indication of a function exhibited by suppressing the expression of SREBP1c and/or MTP and/or PPARγ.

20. A method for preventing or improving a bone strength decrease in a subject in need of the prevention or improvement of bone strength decrease, comprising administering a composition comprising an effective amount of ornithine to the subject.

21. The method according to claim 20, wherein the bone strength decrease is caused by at least one selected from menopause, immobilization osteoporosis, disuse osteoporosis, and aging.

22. The method according to claim 20, wherein the bone strength decrease is caused by bone density decrease or bone quality decrease.

23. The method according to claim 22, wherein the bone quality decrease is caused by bone metabolism decrease.

24. The method according to any one of claims 20 to 23, wherein the prevention or improvement of bone strength decrease is achieved by the promotion of osteoblast differentiation and/or suppression of osteoclast differentiation.

25. The method according to any one of claims 20 to 24, wherein the administration subject is in further need of the prevention or improvement of musculoskeletal function decrease.

26. The method according to claim 20, wherein the administration subject is a subject with a decreased BMP-2 signal.

27. The method according to claim 26, wherein the subject with a decreased BMP-2 signal is a postmenopausal woman, an osteoporosis patient, or an elderly person.

28. A method for inducing osteocalcin expression in a subject in need of the induction of osteocalcin expression, comprising administering a composition comprising an effective amount of ornithine to the subject.

29. A method for preventing or improving changes in physical condition due to changes in postmenopausal hormone balance in a subject in need of the prevention or improvement of changes in physical condition due to changes in postmenopausal hormone balance, comprising administering a composition comprising an effective amount of ornithine to the subject.

30. The method according to claim 29, wherein the change in the physical condition is caused by bone strength decrease and/or liver weight increase.

31. The method according to any one of claims 20 to 30, wherein the composition is orally administered.

32. A method for suppressing the expression of MuRF1 and/or Atg3 in a subject in need of the suppression of the expression of MuRF1 and/or Atg3, comprising administering a composition comprising an effective amount of ornithine to the subject.

33. A method for suppressing the expression of SREBP1c and/or MTP and/or PPARγ in a subject in need of the suppression of the expression of SREBP1c and/or MTP and/or PPARγ, comprising administering a composition comprising an effective amount of ornithine to the subject.

34. A composition comprising ornithine for use in the prevention or improvement of bone strength decrease.

35. The composition according to claim 34, wherein the bone strength decrease is caused by at least one selected from menopause, immobilization osteoporosis, disuse osteoporosis, and aging.

36. The composition according to claim 34, wherein the bone strength decrease is caused by bone density decrease or bone quality decrease.

37. The composition according to claim 36, wherein the bone quality decrease is caused by bone metabolism decrease.

38. The composition according to any one of claims 34 to 37, wherein the prevention or improvement of bone strength decrease is caused by the promotion of osteoblast differentiation and/or the suppression of osteoclast differentiation.

39. The composition according to any one of claims 34 to 38, wherein the composition is further for use in the prevention or improvement of musculoskeletal function decrease.

40. The composition according to claim 34, wherein the prevention or improvement of bone strength decrease is prevention or improvement of bone strength decrease in a subject with a decreased BMP-2 signal.

41. The composition according to claim 40, wherein the subject with a decreased BMP-2 signal is a postmenopausal woman, an osteoporosis patient, or an elderly person.

42. A composition comprising ornithine for use in the induction of osteocalcin expression.

43. A composition comprising ornithine for use in the prevention or improvement of changes in physical condition due to changes in postmenopausal hormone balance.

44. The composition according to claim 43, wherein the change in physical condition is caused by bone strength decrease and/or liver weight increase.

45. The composition according to any one of claims 34 to 44, wherein the composition is a food.

46. A composition comprising ornithine for use in the suppression of the expression of MuRFl and/or Atg3.

47. A composition comprising ornithine for use in the suppression of the expression of SREBPlc and/or MTP and/or PPARγ.

48. Use of ornithine for the production of a composition for preventing or improving bone strength decrease.

49. The use according to claim 48, wherein the bone strength decrease is caused by at least one selected from menopause, immobilization osteoporosis, disuse osteoporosis, and aging.

50. The use according to claim 48, wherein the bone strength decrease is caused by bone density decrease or bone quality decrease.

51. The use according to claim 50, wherein the bone quality decrease is caused by bone metabolism decrease.

52. The use according to any one of claims 48 to 51, wherein the composition is an osteoblast differentiation promoter and/or an osteoclast differentiation inhibitor.

53. The use according to any one of claims 48 to 52, wherein the composition is further for use in the prevention or improvement of musculoskeletal function decrease.

54. The use according to claim 48, wherein the prevention or improvement of bone strength decrease is prevention or improvement of bone strength decrease in a subject with a decreased BMP-2 signal.

55. The use according to claim 54, wherein the subject with a decreased BMP-2 signal is a postmenopausal woman, an osteoporosis patient, or an elderly person.

56. Use of ornithine for the production of a composition for inducing osteocalcin expression.

57. Use of ornithine for the production of a composition for preventing or improving changes in physical condition due to changes in postmenopausal hormone balance.

58. The use according to claim 57, wherein the change in physical condition is caused by bone strength decrease and/or liver weight increase.

59. The use according to any one of claims 48 to 58, wherein the composition is a food.

60. Use of ornithine for the production of a composition for suppressing the expression of MuRF1 and/or Atg3.

61. Use of ornithine for the production of a composition for suppressing the expression of SREBP1c and/or MTP and/or PPARγ.
